# EUROPEAN PATENT APPLICATION

(11) **EP 4 767 944 A1**
(43) Date of publication of application: **01.07.2026**
(21) Application number: 24307328.5
(22) Date of filing: 28.12.2024
(51) Int. Cl.: A61B 5/367, A61B 5/00, A61B 5/287, G16H 50/20

(54) **COMPUTER DEVICE FOR ANALYSIS OF ELECTROGRAMS AND METHOD**

(71) Applicant: Substrate HD, 13006 Marseille (FR)
(72) Inventor: DEMARCY, Thomas, 13006 MARSEILLE (FR); SILVA, Santiago, 13006 MARSEILLE (FR)
(74) Representative: Plasseraud IP

(57) **Abstract**

A computer device for analysis of electrograms comprises a data storage (114) arranged to receive electrogram signals each originating from one of a plurality of electrodes of a catheter and catheter electrode distance data, a random convolutional kernel-based feature extractor arranged to receive electrogram signals associated with the electrodes of a catheter for a given timecode and to return an electrode feature vector for each electrogram signal, and an input generator arranged to receive the electrogram feature vectors output by said random convolutional kernel-based feature extractor and catheter electrode distance data relating to the catheter from which these electrogram signals originate, to associate each electrode with at least the three closest electrodes of the catheter based on said catheter electrode distance data, and to return an input vector in which the electrode feature vectors of each electrode is grouped with the electrode feature vectors of its associated electrodes and the corresponding catheter electrode distance data, a gradient-boosted based machine learning module trained on data comprising sets of input vectors labelled with a value indicating for each electrode if its electrogram signal exhibits spatiotemporal dispersion. The computer device is arranged to receive electrogram signals associated and electrode distance date associated with a timecode, to feed them to said input generator, to use the resulting input vectors as input for said gradient-boosted based machine learning module, and to return a dispersion value for each electrode.

## Description

### Technical Field

The field of atrial fibrillation has been rapidly developing in the past 5 years.

### Background

The detection of cardiac areas that promote atrial fibrillation includes both off-line computing devices using software such as Topera and CardioInsight, and real-time computing devices using software such as CARTO.

The Topera software aims to reconstruct the electrical activation of the atria of the heart. Signal acquisition is carried out using a "basket catheter" (a catheter that is deployed over the entire atrium). The analysis is performed in a delayed time, more than 2 minutes after the start of the acquisition. This solution is inconvenient because this type of catheter is difficult to insert and because the contact of the electrodes is not guaranteed. The analysis duration is very long, and the reconstruction does not allow high-definition maps to be obtained, and even fails in the case of complex electrical activation (which represents 70% of atrial fibrillation cases) due to an overly simplistic reconstruction.

The CardioInsight software aims to reconstruct the electrical activity of the heart using a multi-electrode electrocardiogram measurement vest on the patient's skin. The patient wears the vest before the operation, the data is analyzed and accessible during the operation. The analysis is done in delayed time (more than 15 minutes) after the beginning of the acquisition. This solution has the disadvantage of a very long computation time, which imposes a deferred time making it difficult to set up (the patient is required to come a few days ahead and wear the vest so that the data can be extracted before the operation), and a very high cost. In addition, the reconstruction does not allow high-definition maps because the measurements are set up too far apart, and fails in the case of complex electrical activation because of an overly simplistic reconstruction.

The CARTO software published by Biosense & Webster allows the implementation of algorithms to detect cardiac arrhythmias.

The CFAE (Complex Fractionated Atrial Electrograms) algorithm calculates the number of deflection points (variation of the sign of the derivative) in the signals and produces color maps in real-time. The practitioner then interprets these complex maps to determine places of interest. This algorithm is not very specific and relatively simple.

The Ensite software published by Abbott Laboratories allows the implementation of algorithms to detect cardiac arrhythmias.

The Ripple algorithm (Ripple Mapping) is a module of the CARTO software that allows to reproduce the electrical activity of the atria after a first catheter passage. Both the amplitude and the propagation of electrical waves are made visible through this module. This results in maps that are extremely complex for the practitioner to understand. While this may work in simple cases, the analysis fails in the case of complex electrical activation because the maps are too difficult to interpret.

The Applicant has long used a solution which differs from all existing solutions in that it relies on the determination of spatiotemporal dispersion in electrograms. This measurement was discovered and discussed in the article by Seitz et al. "AF Ablation Guided by Spatiotemporal Electrogram Dispersion Without Pulmonary Vein Isolation: A Wholly Patient-Tailored Approach", J Am Coll Cardiol. 2017 Jan, 69 (3) 303-321, https://doi.org/10.1016/j.jacc.2016.10.065 and was the object of a patent family based on French patent application FR1463232.

The Applicant followed this effort with an improved system and filed a patent family based on French patent application FR1852850 which disclosed using two interlinked methods in order to perform real-time detection of cardiac areas that promote atrial fibrillation. Embodiments of this application use a classifier arranged to apply two classification models to electrogram data. One model is faster to apply and less precise than the other, such that it is used to alert the practitioner of the potential interest of a cardiac region.

### Summary

The further works of the Applicant led to filing a patent family based on patent application FR2101234 which discloses a device for determining the presence of spatiotemporal dispersion in electrograms by combining a gradient boosted tree and a convolutional neural network.

In all of the Applicant's inventions, the spatiotemporal dispersion is obtained by comparing the signals of pairs of electrodes. However, this method is rigid as its training is complicated when introducing new types of catheter which have a differing number of electrodes, or when the spatial distribution of the electrodes themselves is not sufficiently similar between different types of catheters.

The invention aims at improving the situation.

According to a first aspect, a computer device computer device for analysis of electrograms is provided, which comprises a data storage arranged to receive electrogram signals each originating from one of a plurality of electrodes of a catheter and catheter electrode distance data, an input generator arranged to receive the electrogram signals with the electrodes of a catheter for a given timecode and catheter electrode distance data relating to the catheter from which these electrogram signals originate, and to return an ordered set of time series in which the time series represent a sampling of the electrogram signals, and their order is derived from the catheter electrode distance data, and a deep learning dispersion module comprising a convolutional neural network arranged to receive an ordered set of time series and to return a vector of dispersion values for the electrodes which these electrogram signals from which the ordered set of time series are derived originate, said deep learning dispersion module comprising at least two convolution blocks in series and comprising each at least to convolution modules also in series with their respective convolution block and which each perform depth wise and point wise convolution, and a head block for receiving the output of the last convolution block and comprising at least one average pooling block arranged to reduce the dimension of the output of the last convolution block and to return a set of dispersion values for the electrodes which these electrogram signals from which the ordered set of time series are derived originate.

This device is advantageous because it allows a spatiotemporal measurement to be made which takes into account the 3D repartition of the electrodes and it is inherently parallelizable. These features allow to reliably use a convolutional neural network architecture which works directly on the electrogram signals, hence simplifying the signal processing.

In various embodiments, the computer device may present one or more of the following features: According to a second aspect, a method for determining spatiotemporal dispersion in electrograms is provided. This computer implemented method for analyzing electrogram signals and corresponding electrode distance data comprises the following operations:
- the device operates in real-time on electrogram signals and the electrode distance data acquired in real-time,
- at least one of the convolution blocks comprises a shortcut connection to a lower convolution block,
- the head block comprises an average pooling block and the deep learning dispersion module comprises a global average pooling block downstream of the head block, and
- the device is further arranged to determine a color associated to the spatiotemporal dispersion values for each electrogram signal, the computer device further comprising a display arranged to output, for each electrode, the color associated to the spatiotemporal dispersion value determined for the corresponding electrogram signal.

According to a second aspect, a computer implemented method for analyzing electrogram signals and corresponding electrode distance data is provided, which comprises the following operations:
a) Receiving electrogram signals with the electrodes of a catheter for a given timecode and catheter electrode distance data relating to the catheter from which these electrogram signals originate,
b) Deriving an ordered set of time series in which the time series represent a sampling of the electrogram signals, and their order is derived from the catheter electrode distance data,
c) Applying a deep learning dispersion module comprising a convolutional neural network arranged to receive an ordered set of time series and to return a vector of dispersion values for the electrodes which these electrogram signals from which the ordered set of time series are derived originate, said deep learning dispersion module comprising at least two convolution blocks in series and comprising each at least to convolution modules also in series with their respective convolution block and which each perform depth wise and point wise convolution, and a head block for receiving the output of the last convolution block and comprising at least one average pooling block arranged to reduce the dimension of the output of the last convolution block and to return a set of dispersion values for the electrodes which these electrogram signals from which the ordered set of time series are derived originate.

In various embodiments, this method may present one or more of the following features:
- the method is performed operated in real-time on electrogram signals and the electrode distance data acquired in real-time,
- at least one of the convolution blocks of the deep learning dispersion module of operation c) comprises a shortcut connection to a lower convolution block,
- the head block of the deep learning dispersion module of operation c) comprises an average pooling block, and the deep learning dispersion module of operation c) comprises a global average pooling block downstream of the head block, and
- the method further comprises d) determining a color associated to the spatiotemporal dispersion values for each electrogram signal, and outputting, for each electrode, the color associated to the spatiotemporal dispersion value determined for the corresponding electrogram signal.

According to a third aspect, a computer program is provided. The computer program includes instructions for adapting an apparatus to perform the method of any one of the embodiments of the second aspect.

According to a fourth aspect, a carrier is provided, which contains a computer program that includes instructions for adapting an apparatus to perform the method of any one of the embodiments of the second aspect is provided. In some embodiments, the carrier is one of an electronic signal, optical signal, radio signal, or computer readable storage medium.

According to a fifth aspect, a computer program product is provided, which comprises a non-transitory computer readable medium having stored thereon a computer program according to the third aspect.

Other features and advantages of the invention will readily appear in the following description of the drawings, which show exemplary embodiments of the invention and on which:
- Figure 1 shows a general diagram of a computer device according to an embodiment,
- Figure 2 shows a generic view of a computer program used in the embodiment of figure 1,
- Figure 3 shows an exemplary embodiment of a module of figure 2,
- Figure 4 shows a visual return on a display based on spatiotemporal dispersion values, and
- Figure 5 shows a flow chart illustrating a process according to an embodiment.

The drawings and the following description are comprised for the most part of positive and well-defined features. As a result, they are not only useful in understanding the invention, but they can also be used to contribute to its definition, should the need arise.

Persistent atrial fibrillation remains a therapeutic challenge. While an increasingly larger number of patients is eligible for catheter ablation, the optimal ablation strategy for persistent atrial fibrillation remains elusive. STAR-AF2 (Verma A, Jiang C-y, Betts TR et al. "Approaches to catheter ablation for persistent atrial fibrillation". New England Journal of Medicine 2015; 372:1812-1822) concluded that performing additional ablation lesions beyond pulmonary vein isolation (PVI) does not lead to improved long-term outcomes.

By contrast, some observational studies (see Jadidi AS, Lehrmann H, Keyl C et al. "Ablation of persistent atrial fibrillation targeting low-voltage areas with selective activation characteristics", Circulation: Arrhythmia and Electrophysiology 2016;9:e002962 and Seitz J, Bars C, Théodore G et al. "AF ablation guided by spatiotemporal electrogram dispersion without pulmonary vein isolation: a wholly patient-tailored approach", Journal of the American College of Cardiology 2017;69:303-321) have suggested that non-PVI lesions targeting regions harboring electrogram abnormalities are beneficial to patients with persistent atrial fibrillation.

In particular, the latter study showed the results of which supported the application of radiofrequency energy in atrial regions exhibiting the dispersion of multipolar electrograms. Other studies (Narayan SM, Krummen DE, Shivkumar K, Clopton P, Rappel W-J, Miller JM. "Treatment of atrial fibrillation by the ablation of localized sources: CONFIRM (Conventional Ablation for Atrial Fibrillation With or Without Focal Impulse and Rotor Modulation) trial", Journal of the American College of Cardiology 2012;60:628-636) instead performed advanced signal analysis and provided a mechanistic-based display to guide operators towards atrial fibrillation drivers.

Regardless of the electrogram abnormalities or analyses, there are large differences in experience and stage in the learning curve between centers attempting to implement similar electrogram-based mapping approaches.

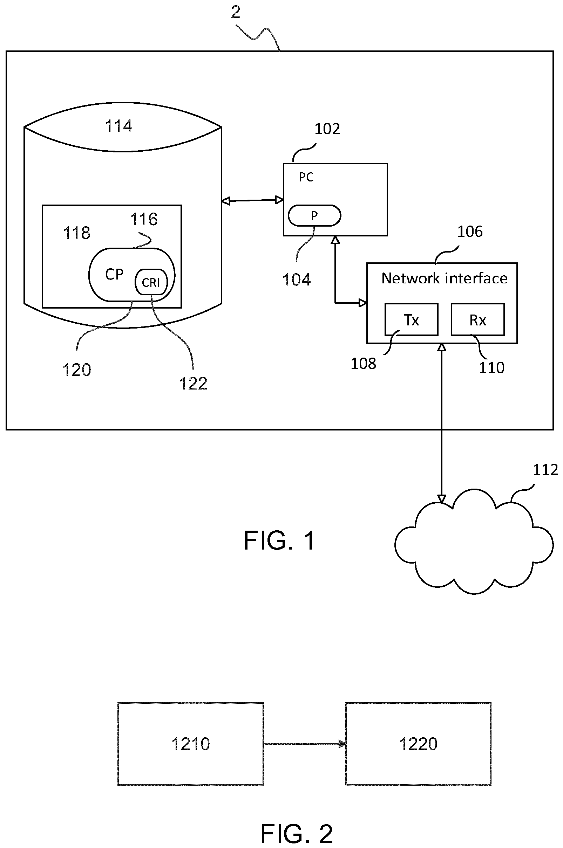

Figure 1 shows a general diagram on an embodiment of the computer device according to the invention.

The computer device shown on Figure 1 as a block diagram of an apparatus 2 (*e.g.,* a network node, connected device, and the like), according to an embodiment. As shown in Figure 1 the apparatus may comprise: processing circuitry (PC) 102, which may include one or more processors (P) 104; a network interface 106 comprising a transmitter (Tx) 108 and a receiver (Rx) 110 for enabling the apparatus to transmit data to and receive data from other computing devices connected to a network 112 (e.g., an Internet Protocol (IP) network) to which network interface 106 is connected; and a local storage unit (a.k.a., "data storage system") 114, which may include one or more non-volatile storage devices and/or one or more volatile storage devices.

In embodiments where PC 102 includes a programmable processor, a computer program product (CPP) 116 may be provided. CPP 116 includes a computer readable medium (CRM) 118 storing a computer program (CP) 120 comprising computer readable instructions (CRI) 122. CRM 118 may be a non-transitory computer readable medium, such as, magnetic media (e.g., a hard disk), optical media, memory devices (e.g., random access memory, flash memory), and the like.

Processors 104 include any means known for performing automated calculus, such as CPUs, GPUs, CPUs and/or GPUs grids, remote calculus grids, specifically configured FPGAs, specifically configured ASICs, specialized chips such as SOCs or NOCs, AI specialized chips, etc.

In an embodiment, data storage 114 stores electrogram signals (for example after they have been digitalized and split at regular time intervals, such that all electrogram signals have the same length, e.g., 3 seconds) along with catheter electrode distance data. The electrogram signals may be based on real-time acquisition of electrograms by a catheter during a procedure, or they may be based on differed-time. The electrogram signals are the result of the sensing by the electrodes. The same applies to the catheter electrode distance data. The catheter electrode distance data may also be fixed, *e.g.* be based on theoretical or manufacturer provided distance data between the electrodes of a given catheter model. The catheter electrode distance data may be explicit (*e.g*. comprise location information or distance values) or implicit (*e.g.* a reference attached to the corresponding electrogram signal may be used to identify the corresponding acquisition electrode and provide electrode distance information in view of the catheter model which was used for the acquisition). As will appear below, the electrogram signals are transformed into electrogram time series. The electrode time series are also stored in data storage 114. The database may further contain a training set of electrogram signals, corresponding catheter electrode distance data, each labelled with a value indicating whether they exhibit spatiotemporal dispersion, *i.e.,* whether they are associated with atrial fibrillation or not. In the example described herein, the Applicant has established a database of almost 500000 such elements which form its training dataset (almost 21h30m of signals). Electrogram signals may also be post-treated in order to be added to the training dataset for retraining or continuous training. The results return by apparatus 2 may be used by the practitioner to mark (or otherwise have the associated position returned in an automatic manner) the corresponding locations on a patient's heart, which leads to a map of a heart with zones to be treated in view of atrial fibrillation represented as dots. In some embodiments, this map can be generated automatically based on the apparatus 2 outputs.

In the example described herein, the data storage 114 may be realized in any way suitable, that is by means of a hard disk drive, a solid-state drive, a flash memory, a memory embedded in a processor, a distant storage accessible in the cloud, etc. Data storage 114 may also store any transitory data which may be generated in the course of executing the invention, as well as maps resulting from the operation of the apparatus 2, possibly combined with practitioner made annotations.

Figure 2 shows an exemplary schematic of computer program 120.

The computer program (CP) 120 comprises two main modules which work hand-in-hand:
- An input generator module 1210, and
- A deep learning dispersion module 1220.

In the example described herein, the input generator 1210 receives electrogram signals as an input. The input electrogram signals are associated with electrode identifiers from which they were obtained, as well as electrode distance data, which may be explicit or implicit as described above. The input generator 1210 outputs, for each series of input electrogram signals, an ordered set of time series. In this ordered set of time series, the input electrogram signals are sampled according to a chosen duration, which is that of the time series used to train the deep learning dispersion module 1220, and an order, which is determined based on the electrode distance data. In other words, the order of the time series in the ordered time series carries the electrode distance information in the processing by the deep learning dispersion module 1220.

In different embodiments, the input generator 1210 may modify more or less extensively the input electrogram signals.

In its simplest version, the input generator 1210 merely acts as a wrapper for the deep learning dispersion module 1220. In other words, the input generator 1210 uses the input electrogram signals and the corresponding electrode distance data, and it packages into a vector or list which contains the ordered time series, in the same format which has been used for the deep learning dispersion module 1220.

In a more complex embodiment, the input generator 1210 may perform several operations on the input electrode signals before returning the ordered time series. For example, the input generator 1210 may filter the input electrogram signals in order to improve the processing by the deep learning dispersion module 1220, *e.g.* to correct sampling artefacts or to change the sampling rate, by downsampling or upsampling. The input generator 1210 may also process the input electrogram signals in order to harmonize the ordered time series, for example when the input electrogram signals have a different length or when their length exceeds that of the ordered time series.

Examples of filtering include but are not limited to:
- applying a low-pass filter, which can effectively reduce high-frequency noise,
- applying a high-pass filter, which can eliminate low-frequency drift, ensuring that the signals accurately reflect the underlying physiological phenomena,
- applying wavelet transforms, which can help in decomposing the signal into different frequency components, allowing for more targeted filtering and noise reduction,
- applying a notch filter, which can be effective in removing powerline artifacts from electrogram signals, which are often introduced by electrical interference at frequencies such as 50 Hz or 60 Hz, depending on the region,
- performing a wavelet decomposition using - for example - the Daubechies wavelet which is an effective method for noise reduction in cardiac signals, as it allows for the analysis of the signal at multiple scales,
- applying simple filters, such as moving average or exponential filter, which can smooth the signal by averaging neighboring samples, which can help reduce random noise.

In some embodiments, the input generator 1210 could be considered as a mere input mode of the deep learning dispersion module 1220.

The deep learning dispersion module 1220 is an XceptionTime model, which is a CNN (convolutional neural network). This model is described in the article by F. Chollet, "Xception: Deep Learning with Depthwise Separable Convolutions", 2017 IEEE 7 Conference on Computer Vision and Pattern Recognition (CVPR), Honolulu, HI, USA, 2017, 8 pp. 1800-1807, doi: 10.1109/CVPR.2017.195.

In the past, the Applicant already tried to use CNN models. However, as described in FR1852850, it was necessary to combine the CNN with a classic machine learning approach to get the best results. Furthermore, the training of CNNs is resource intensive, and this limited the amount of data which the Applicant managed to use.

By searching and developing its solutions, the Applicant discovered that the addition of the electrode distance information in the ordered time series allowed to use a CNN by itself. Further searches allowed the Applicant to discover that the XceptionTime model was particularly effective for determining dispersion. Indeed, At the core of the XceptionTime model is a series of blocks, called XceptionBlocks, each constructed to perform feature extraction through hierarchical processing stages. Each XceptionBlock comprises multiple Xception modules, which are the fundamental building units responsible for capturing temporal patterns at various scales.

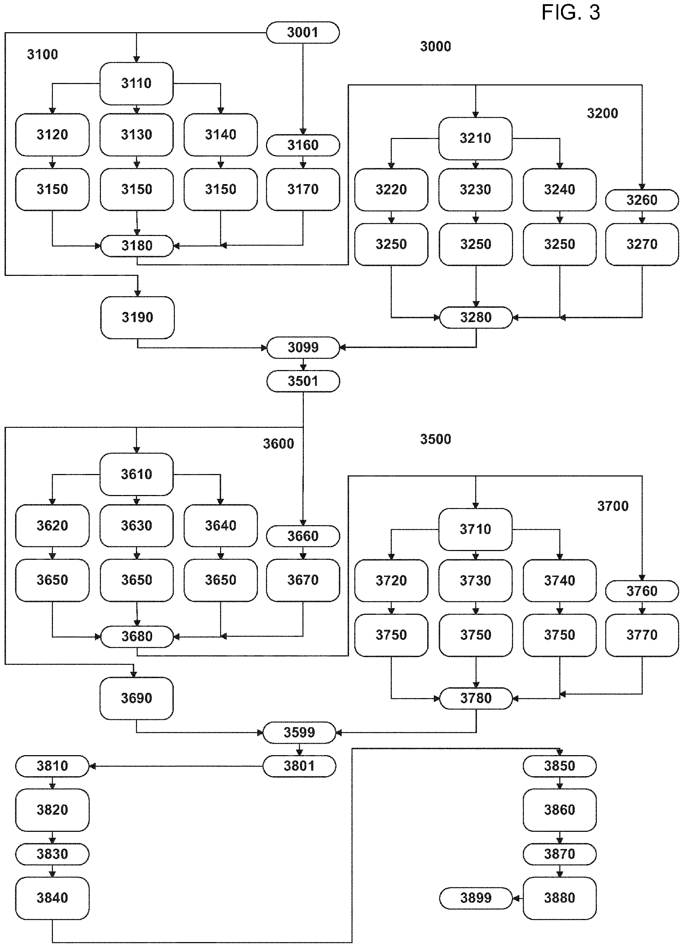

Figure 3 shows an exemplary embodiment of an XceptionTime model which has been used by the Applicant. In this embodiment, the deep learning dispersion module 1220 comprises two XceptionBlocks 3000 and 3500. The goal of the XceptionBlocks 3000 and 3500 is to use a multiscale approach to learn both long-term dependencies and fine-grained temporal patterns within the data. To that end, each XceptionBlock comprises at least two Xception modules, which are the building units responsible for capturing temporal patterns at various scales.

In the example shown on figure 3, the XceptionBlock 3000 comprises two Xception modules 3100 and 3200. Each of them begins with a bottleneck layer, implemented as a one-dimensional convolutional layer 3110 (respectively 3210). This layer serves to reduce the dimensionality of the input data. The ordered time series are received in an input 3001 and fed to the bottleneck layer 3110. In the example described herein, the ordered time series are fed as a 1*5*1500 element, and the bottleneck layer has 16*5*1 dimensions.

Following the bottleneck layer 3110, each Xception module incorporates a series of separable convolutions, implemented as convolutional layers 3120, 3130 and 3140 (respectively 3220, 3230 and 3240).

These convolutions are depth wise separable, decomposing standard convolution operations into depth wise and pointwise convolutions, significantly reducing the number of parameters compared to standard convolutions. The depth wise convolutions use varying kernel sizes, here kernels of sizes 39, 19, and 9 to capture temporal features at multiple resolutions. This multiscale approach allows the model to learn both long term dependencies and fine-grained temporal patterns within the data. After the depth wise convolutions, the point wise convolution is performed by a convolution layer 3150 (respectively 3250) with a 16*16*1 dimension.

To further refine the feature extraction process, each XceptionModule includes a MaxConvPool component. This component combines a max pooling 3160 (respectively 3260) with a one-dimensional convolution 3170 (respectively 3270) with a 16*5*1 dimension, effectively summarizing local temporal features while preserving the essential characteristics of the input sequence. The outputs from the separable convolutions and the MaxConvPool are then concatenated in a concatenator 3180 (respectively 3280), aggregating diverse feature representations and enriching the overall capacity of the model to capture complex temporal dynamics.

Shortcut connections are integrated to facilitate the flow of gradients during training, thus addressing the problem of vanishing gradients. These shortcuts 3190 employ a one-dimensional convolution of with a dimension 128*5*1 coupled with a batch normalization layer of dimension 128 to ensure that the dimensions of the inputs and outputs are compatible for element wise addition. The model employs an additive merging 3099, combining the outputs of the main convolutional paths with the shortcut paths. This residual connection strategy enables the model to learn identity mappings more effectively, contributing to faster convergence and improved performance.

The XceptionBlock 3500 is similar to the XceptionBlock 3000. Hence, the same last two digits of reference numbers will be used for identical elements. XceptionBlock 3500 comprises two Xception modules 3600 and 3700. The input of XceptionBlock 3500 is the output of additive merging 3099, which passes it through a ReLu 3501 (or Rectified Linear Unit), which is connected to the bottleneck layer 3610.

After the XceptionBlock 3500, the deep learning dispersion module 1220 comprises a head block 3800 for the final stages of processing. Head block 3800 comprises an average pooling portion which may be adaptative in some embodiments.

Average pooling portion receives the output of the XceptionBlock 3500 after a ReLu 3801, in an average pooling block 3810. The average pooling block 3810 layer reduces the temporal dimensions of the feature maps to a fixed size of 50, allowing the model to handle input sequences of varying lengths while maintaining a consistent output dimension. This pooling operation retains the most significant temporal features by averaging over the input sequence, effectively summarizing the information content. The average pooling reduces the feature maps over the temporal dimension.

It is followed by a convolution layer 3820 with a dimension 256*512*1 coupled with a batch normalization layer of dimension 256, which reduces the channel size from 512 to 256. This is repeated with a ReLu 3830 and another convolution layer 3840 with a dimension 128*256*1 coupled with a batch normalization layer of dimension 128, which reduces the channel size from 256 to 128. This sequence facilitates hierarchical feature distillation, refining the features extracted by the earlier layers and preparing them for the final classification task.

The head block 3800 is followed by a global pooling portion which reduces each feature map to a single value by averaging over the temporal dimension. This operation results in a compact feature vector that encapsulates the learned representations from the entire input sequence.

The global pooling portion begins with a ReLu 3850 feeding a convolution layer 3860 with a dimension 2* 128* 1 coupled with a batch normalization layer of dimension 2, which reduces the channel size from 128 to 2. This is followed by a ReLu 3870 which feeds a global average pooling block 3870. The global average pooling averages the feature maps over the temporal dimension, creating a fixed-size compact feature vector.

The global average pooling block 3870 is followed by a flattening operation 3880, reshaping the output to a format suitable for classification. The final output 3899 from the global pooling portion directly serves as the input for the classification decision, acting directly as a classifier. The output 3899 is a dispersion value for each of the electrodes.

The output of the deep learning dispersion module 1220 is, for each order time series, a value reflecting the spatiotemporal dispersion of the corresponding electrogram signals. As mentioned above, the deep learning dispersion module 1220 in the example herein was trained on over 500000 input vectors which were each labelled with a spatiotemporal dispersion value, and tested on more than 110000 input vectors.

The above describes a specific realization of the Xception Time model in the context of the invention. However, this model may be adapted in various manners while providing satisfactory results. For example, and a non-limitative manner:
- the shortcut connections may be partly or fully omitted,
- there can be three of more XceptionBlocks,
- the XceptionBlocks can comprise three or more Xception modules,
- the dimensions of the convolution layers for depth wise and point wise convolutions can be varied,
- the XceptionBlocks can be slightly different between them, *e.g*. some may include shortcut connections while others do not, or the dimensions of the convolution layers can be made to vary,
- the Xception modules can be slightly different between them within a given XceptionBlock, for example regarding the kernel sizes of the depth wise convolution layers,
- the number of kernels sizes of the depth wise convolution layers may also differ from those described here,
- the head block may comprise a single averaging pooling block, etc.

In some embodiments, the CRI 122 of CP 120 is configured such that when executed by PC 102, the CRI 122 causes the apparatus 2 to perform steps described herein (e.g., steps described herein with reference to the block diagrams). In other embodiments, the apparatus may be configured to perform steps described herein without the need for code. That is, for example, PC 102 may consist merely of one or more ASICs. Hence, the features of the embodiments described herein may be implemented in hardware and/or software.

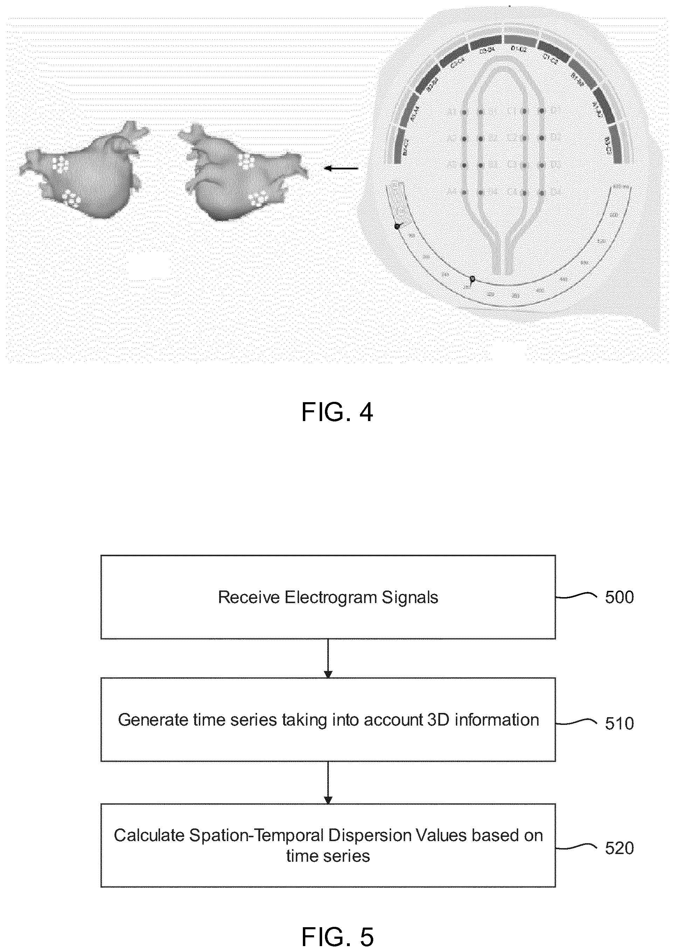

Based on the results of the spatiotemporal dispersion value, the apparatus 2 may further determine colors based on the prediction array. More precisely, this determination can be based by using prediction arrays associated with consecutive timecodes. If it is determined that the atrial fibrillation of the patient is slow, two prediction arrays corresponding to consecutive timecodes can be averaged, and the values of this array be associated as follows: blue if the value is less than 0.35, orange if the value is comprised between 0.35 and 0.65, and else red. If. If it is determined that the atrial fibrillation of the patient is fast, four prediction arrays corresponding to consecutive timecodes can be averaged, and the values of this array be associated as follows: blue if the value is less than 0.5, orange if the value is comprised between 0.5 and 0.8, and else red. The determination of whether the atrial fibrillation is fast or slow is a practitioner decision, which he inputs in the device. This allows to provide a visual return on a display as shown on Figure 4, which may be used by the practitioner to tag automatically, manually or semi-manually the heart region.

Figure 5 shows an exemplary schematic block diagram of the operation of apparatus 2 according to an embodiment. The apparatus 2 includes the modules represented on Figure 2, each of which is implemented in software. These modules provide the functionality of apparatus 2 described herein (e.g., steps described herein).

In a first operation 500, a set of electrogram signals are obtained from electrodes of a catheter and associated with the same timecode.
are fed to the input generator 1210. These electrogram signals are accompanied by electrode distance data which match the same timecode for these electrodes. The result is an o, with one electrode feature vector per originating electrogram signal in the input set. It will be understood that the output does not need to be a vector *per se.* It may be provided in any form as long as the electrode feature vectors are recoverable and separable from one another.

After operation 500, the set of electrogram signals are fed, along with the electrode distance data to the first module, that is the input generator 1120, in an operation 510. As described above, the input generator 1220 provides, an ordered set of time series.

Finally, in an operation 520, the ordered time series are sent to the second module, that is the deep learning dispersion module 1220 in order to calculate the spatiotemporal dispersion value of each electrode signal.

The tests performed by the Applicant have shown the following results:
VX1 (original implementation): precision 0.61 / recall 0.74 / F1 0.67

Embodiment of figure 1: precision 0.65 / recall 0.78 / F1 0.71

The invention thus clearly is an improvement over the implementation of French patent application FR1852850.

While various embodiments of the present disclosure are described herein, it should be understood that they have been presented by way of example only, and not limitation. Thus, the breadth and scope of the present disclosure should not be limited by any of the above-described exemplary embodiments. Moreover, any combination of the above-described elements in all possible variations thereof is encompassed by the disclosure unless otherwise indicated herein or otherwise clearly contradicted by context.

Additionally, while the processes described above and illustrated in the drawings are shown as a sequence of steps, this was done solely for the sake of illustration. Accordingly, it is contemplated that some steps may be added, some steps may be omitted, the order of the steps may be rearranged, and some steps may be performed in parallel.

Some embodiments concern a computer-implemented method for identifying a cardiac region of interest:
Embodiment X. A computer-implemented method for identifying a cardiac region of interest, the method comprising:
   a) receiving electrogram signals with the electrodes of a catheter for a given timecode and catheter electrode distance data relating to the catheter from which these electrogram signals originate;
   b) deriving an ordered set of time series in which the time series represent a sampling of the received electrogram signals, and their order is derived from the received catheter electrode distance data; and
   c) applying a deep learning (DL) dispersion model comprising a convolutional neural network (CNN), wherein the CNN receives the derived ordered set of time series and returns a vector of spatiotemporal dispersion values for the electrodes which these electrogram signals from which the ordered set of time series are derived originate, wherein the returned vector of spatiotemporal dispersion values is used as part of identifying a cardiac region of interest.
Embodiment X1. The computer-implemented method of embodiment X, wherein the CNN of the DL dispersion model includes:
   - at least two convolution blocks in series;
   - each at least two convolution modules in series with their respective convolution block and which each perform depth wise and point wise convolution; and
   - a head block for receiving the output of the last convolution block and comprising at least one average pooling block arranged to reduce the dimension of the output of the last convolution block and to return a set of dispersion values for the electrodes which these electrogram signals from which the ordered set of time series are derived originate.
Embodiment X2. The computer-implemented method of embodiment X, wherein the electrogram signals and the electrode distance data are received in real-time.
Embodiment X3. The computer-implemented method of embodiment X, wherein the identified cardiac region of interest includes the region of a heart that contains atrial tachycardia (AT).

Some embodiments concern a computing device:
Embodiment Y. A computing device comprising:
   processing circuitry; and
   a memory containing instructions executable by the processing circuitry for identifying a cardiac region of interest, the computing device operative to:
      d) receive electrogram signals with the electrodes of a catheter for a given timecode and catheter electrode distance data relating to the catheter from which these electrogram signals originate;
      e) derive an ordered set of time series in which the time series represent a sampling of the received electrogram signals, and their order is derived from the received catheter electrode distance data; and
      f) apply a deep learning (DL) dispersion model comprising a convolutional neural network (CNN), wherein the CNN receives the derived ordered set of time series and returns a vector of spatiotemporal dispersion values for the electrodes which these electrogram signals from which the ordered set of time series are derived originate, wherein the returned vector of spatiotemporal dispersion values is used as part of identifying a cardiac region of interest.

In this computing device, the generated spatiotemporal dispersion values are used for identifying a cardiac region of interest.

In this computing device, the set of electrogram signals received may be provided in real-time during a procedure.

## Claims

1. A computer device for analysis of electrograms, comprising a data storage (114) arranged to receive electrogram signals each originating from one of a plurality of electrodes of a catheter and catheter electrode distance data,
an input generator (1210) arranged to receive the electrogram signals with the electrodes of a catheter for a given timecode and catheter electrode distance data relating to the catheter from which these electrogram signals originate, and to return an ordered set of time series in which the time series represent a sampling of the electrogram signals, and their order is derived from the catheter electrode distance data,
a deep learning dispersion module (1220) comprising a convolutional neural network arranged to receive an ordered set of time series and to return a vector of dispersion values for the electrodes which these electrogram signals from which the ordered set of time series are derived originate, said deep learning dispersion module (1220) comprising at least two convolution blocks (3000, 3500) in series and comprising each at least to convolution modules (3100, 3200, 3600, 3700) also in series with their respective convolution block and which each perform depth wise and point wise convolution, and a head block (3800) for receiving the output of the last convolution block (3500) and comprising at least one average pooling block arranged to reduce the dimension of the output of the last convolution block (3500) and to return a set of dispersion values for the electrodes which these electrogram signals from which the ordered set of time series are derived originate.

2. Computer device according to claim 1, in which the computer device operates in real-time on electrogram signals and the electrode distance data acquired in real-time.

3. Computer device according to claim 1 or 2, in which at least one of the convolution blocks (3000) comprises a shortcut connection (3190) to a lower convolution block (3500).

4. Computer device according to one of the preceding claims, in which the head block (3800) comprises an average pooling block (3810), and the deep learning dispersion module (1220) comprises a global average pooling block (3870) downstream of the head block (3800).

5. Computer device according to one of the preceding claims, further arranged to determine a color associated to the spatiotemporal dispersion values for each electrogram signal, the computer device further comprising a display arranged to output, for each electrode, the color associated to the spatiotemporal dispersion value determined for the corresponding electrogram signal.

6. Computer implemented method for analyzing electrogram signals and corresponding electrode distance data comprising the following operations:
a) Receiving (400) electrogram signals with the electrodes of a catheter for a given timecode and catheter electrode distance data relating to the catheter from which these electrogram signals originate,
b) Deriving (410) an ordered set of time series in which the time series represent a sampling of the electrogram signals, and their order is derived from the catheter electrode distance data,
c) Applying (420) a deep learning dispersion module (1220) comprising a convolutional neural network arranged to receive an ordered set of time series and to return a vector of dispersion values for the electrodes which these electrogram signals from which the ordered set of time series are derived originate, said deep learning dispersion module (1220) comprising at least two convolution blocks (3000, 3500) in series and comprising each at least to convolution modules (3100, 3200, 3600, 3700) also in series with their respective convolution block and which each perform depth wise and point wise convolution, and a head block (3800) for receiving the output of the last convolution block (3500) and comprising at least one average pooling block arranged to reduce the dimension of the output of the last convolution block (3500) and to return a set of dispersion values for the electrodes which these electrogram signals from which the ordered set of time series are derived originate.

7. Computer implemented method according to claim 6, which is performed operated in real-time on electrogram signals and the electrode distance data acquired in real-time.

8. Computer implemented method according to one of claims 6 and 7, in which at least one of the convolution blocks (3000) of the deep learning dispersion module (1220) of operation c) comprises a shortcut connection (3190) to a lower convolution block (3500).

9. Computer implemented method according to one of claims 6 to 8, in which the head block (3800) of the deep learning dispersion module (1220) of operation c) comprises an average pooling block (3810), and the deep learning dispersion module (1220) of operation c) comprises a global average pooling block (3870) downstream of the head block (3800).

10. Computer implemented method according to one of claims 6 to 9, further comprising d) determining a color associated to the spatiotemporal dispersion values for each electrogram signal, and outputting, for each electrode, the color associated to the spatiotemporal dispersion value determined for the corresponding electrogram signal.

11. A computer program (120) comprising instructions (122) which, when executed by processing circuitry (102), causes the processing circuitry to carry out the methods of any one of claims 6 to 10.

12. A carrier containing a computer program (120) according to claim 11 wherein the carrier comprises one of an electronic signals, optical signal, radio signal or computer readable storage medium.
